# EUROPEAN PATENT SPECIFICATION

(11) **EP 1 793 886 B1**
(45) Date of publication and mention of the grant of the patent: **26.11.2008**
(21) Application number: 05800958.0
(22) Date of filing: 26.09.2005
(51) Int. Cl.: A61M 25/09

(54) **Loop tip wire guide**
Schleifenspitzendrahtführung
Guide-fil terminé par une boucle

(30) Priority: 30.09.2004 US 614756 P
(43) Date of publication of application: 13.06.2007
(73) Proprietor: Wilson-Cook Medical Inc., Winston-Salem, NC 27105-4191 (US)
(72) Inventor: AYALA, Juan, Carlos, Santiago (CL); FOUSHEE, Jason, D., Durham, North Carolina 27713 (US); HARDIN, David, M, Jr., Winston-Salem, NC 27106 (US)
(74) Representative: Garratt, Peter Douglas
(86) International application number: PCT/US2005/034281
(87) International publication number: WO 2006/039217

(56) References cited:
- WO-A-20/04050161
- WO-A-20/04089456
- JP-A- 7 088 191
- US-A1- 2002 010 426
- US-A1- 2002 087 100

## Description

### FIELD OF THE INVENTION

The present invention relates to wire guides used in the placement of medical devices. More specifically, the present invention relates to a wire guide having a loop tip.

### BACKGROUND OF THE INVENTION

Wire guides are elongate flexible members used to provide a path along which another medical device can be moved. The path provided by the wire guide can be used to navigate another medical device, such as a catheter, through a body vessel. The use of wire guides to define such a path is known in the art. Briefly, a wire guide is navigated through a body vessel toward a point of treatment. Once positioned within the vessel, a second medical device, frequently a cannula such as a catheter, is placed over the wire guide and moved along its length toward the point of treatment. Thus, the wire guide provides an established path for placing other devices, eliminating the need for performing delicate navigation procedures for each device passed into the vessel.

During placement of a wire guide, an operator must navigate the wire guide through the vessel(s). Often, the vessel defines a torturous path due to the presence of natural bends and/or curves, or unnatural impediments, such as tumors, build-ups, and/or strictures. The presence of a torturous path may make navigation of a wire guide difficult. For example, the presence of an impediment may block the wire guide from navigating further into the vessel.

The prior art contains many examples of wire guides having straight flexible tips intended to aid in the navigation around such impediment. The presence of a straight flexible tip, however, may in fact make navigation more difficult. For example, upon encountering an impediment, the straight flexible tip may bend toward one of the vessel walls, which may result in unintended contact between the tip and vessel wall. This situation may lead to undesirable effects in the vessel wall. Further, the straight tip may bend and turn back upon itself upon encountering the impediment. This formation of an unstable turn in the wire guide makes further navigation difficult.

### BRIEF SUMMARY OF THE INVENTION

The present invention provides a wire guide capable of manipulating about a tortuous path and/or an impediment. The wire guide comprises a loop at one end. A wire guide according to the present invention comprises an elongate member having a first portion with a first diameter and a second portion with a second diameter. The second diameter is smaller than the first diameter.

The elongate member defines a loop. The elongate member is bent back upon itself to form a loop having an interior space. A closure member closes the loop such that the interior space is closed. Reference is directed in this connection to WO 2004/050161. According to the present invention, the closure member comprises at least one attachment tab, which is bent about a portion of the elongate member.

In one embodiment, the elongate member further comprises an intermediate region defining a taper from the first diameter to the second diameter. Preferably, the loop places a distal end of the wire guide adjacent this intermediate portion. Alternatively, the loop can place the distal end adjacent the second portion.

The second portion can define a portion of the loop, or the entire loop. If present, the intermediate portion can define a portion of the loop.

The loop is resilient and is preferably fixed in overall size. The closure member preferably fixes the distal end relative to another portion of the elongate member. Also preferable, the loop defines a loop width that is greater than the first diameter of the first portion of the elongate member.

In one embodiment, a covering is positioned over at least the closure member. Particularly preferable, the covering is positioned over the closure member and at least the first portion of the elongate member.

### BRIEF DESCRIPTION OF THE DRAWINGS

Figure 1 is a side view of a wire guide not according to the invention.

Figure 2 is a side view of a wire guide not according to the invention.

Figure 3 is a side view of a wire guide not according to the invention.

Figure 4 is an elongate cross-sectional view of a wire guide not according to the invention.

Figure 5 is a side view illustrating a wire guide encountering an impediment in a body vessel.

Figure 6 is a side view illustrating a wire guide encountering a torturous path within a body vessel.

Figure 7 is a side view of a wire guide not according to the invention.

Figure 8 is a side view of a wire guide not according to the invention.

Figure 9 is a side view of a wire guide not according to the invention.

Figure 10 is a side view of a wire guide not according to the invention.

Figure 11 is a side view of a wire guide not according to the invention.

Figure 12 is a side view of a wire guide not according to the invention.

Figure 13 is a side view of a wire guide according to the invention.

Figure 14 is a top-view of the steerable wire-guide of Figure 13 in an unassembled form illustrating an alternate embodiment of a closure member.

Figure 15 is a side-view of the steerable wire-guide of Figure 13.

### DETAILED DESCRIPTION OF THE INVENTION

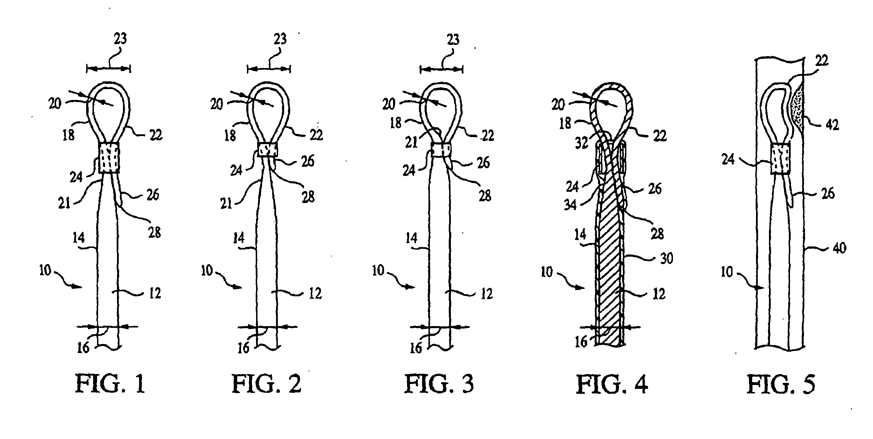

Figure 1 illustrates a wire guide 10 . The wire guide comprises an elongate member 12 having a first portion 14 with a first diameter 16 and a second portion 18 with a second diameter 20. The second diameter 20 is smaller than the first diameter 16. The elongate member 12 has an intermediate portion 21 that defines a taper from the first diameter 16 to the second diameter 20.

The elongate member 12 defines a loop 22. The loop 22 comprises a section of the elongate member 12 bent back upon itself. As illustrated in Figures 1 and 2, the second portion 18 preferably defines the entire loop 22. Alternatively, as illustrated in Figure 3, the second portion 18 can defines only a portion of the loop 22. In this embodiment, the intermediate portion 21 preferably defines at least a portion of the loop 22.

Preferably, as illustrated in the figure, the loop 22 comprises a curvilinear loop forming a generally ovoid shape. Also preferable, the loop 22 has a loop width 23 that is greater than the first diameter 16 of the first portion 14 of the elongate member 12. The term 'loop width' refers to the distance between the two outer most surfaces of the elongate member 12 at the widest portion of the loop 22.

The elongate member 12 has a distal end 26 and a distal tip 28. Preferably, the distal tip 28 tapers from the second diameter 20 to a smaller diameter, and particularly preferably tapers to a point. As illustrated in Figure 1, the loop 22 is preferably formed in a manner that positions the distal end 26 adjacent the intermediate portion 21. Preferably, this placement also positions the distal tip 28 adjacent the intermediate portion 21. Such placements provide a low profile over the portion of the elongate member 12 that has a double width (i.e., two sections of the elongate member 12). Alternatively, as illustrated in Figure 2, the loop 22 can be formed such that the distal end 26 is positioned adjacent the second portion 18 of the elongate member 12.

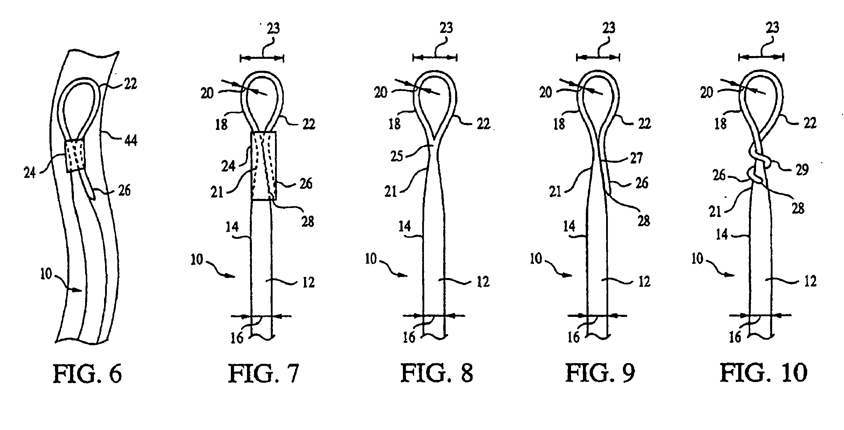

Any method of forming loop 22 is contemplated. A closure member 24 closes the loop 22 such that no opening exists to the interior space of the loop 22. As illustrated in Figure 1, the closure member 24 preferably positions the distal end 26 adjacent another portion of the elongate member 12. Any suitable closure member can be used, including bonds, adhesives, and separate members. Examples of suitable closure members include sutures or other appropriate material tying the two sections together, adhesive bonds and other bonds (such as a solder bond, a welded bond, or a molded bond) and a connector (such as a rivet). As best illustrated in Figures 1 and 7, the closure member 24 preferably is a cannula defining an interior lumen. Two sections of the elongate member are positioned within the cannula to form the loop 22. As shown in Figure 7, the cannula preferably extends over and covers the distal end 26 and distal tip 28. Preferably, the closure member 24 is tightened, such as by crimping, to fix the loop 22 in overall size.

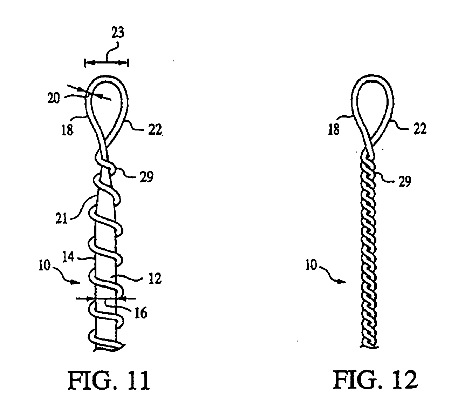

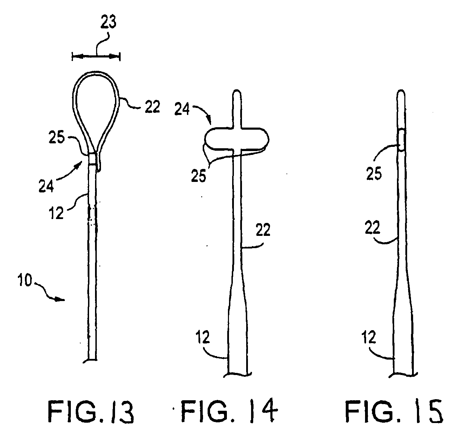

In the example shown in Figure 8, the loop 22 of wire guide 10 is formed by molding two sections of the elongate member together. In another alternate preferred embodiment shown in Figure 9, two sections of the elongate member are welded or soldered together to form loop 22. In the alternate examples shown in Figures 10-12, the loop 22 of wire guide 10 if formed from a coiled wire. More specifically, two sections of the elongate member are wound about each other. Preferably, the distal end 26 is wound such that a low profile is achieved. In yet another alternate preferred embodiment (not shown), the loop 22 and elongate member 12 of wire guide 10 may be formed using laser cutting techniques as are known to those skilled in the art. In the embodiment according to the invention shown in Figures 13-15, the closure member 24 comprises at least one attachment tab 25, which is bent about a portion of the elongate member 12. Optionally, the at least one attachment tab 25 can also be bonded to the elongate member 12 via any method known in the art including, but not limited to, solder, welded or adhesive bonded.

Any suitable material can be used for the elongate member 12, and a variety of suitable materials are known to those skilled in the art. The material chosen need only be biocompatible and able to be formed into the structures described herein. Examples of suitable materials include stainless steel and nitinol. The elongate member 12 may comprise a wire, a tubular member or a sheet of material. Further, the elongate member 12 can be formed of a series of layers, or as a coated core structure. For example, in one embodiment, the elongate member 12 comprises a Nitinol(TM) core with a polytetrafluoroethylene covering.

The closure member 24 can be formed of any suitable material, and need only be biocompatible and capable of maintaining the loop 22 in a closed position. Preferably, the closure member 24 comprises attachment tabs 25 formed of stainless steel or Nitinol(TM). Also preferable, the closure member 24 is able to maintain a tightened position on the elongate member 12 upon application of a suitable force, such as by applying a crimping workload to the closure member 24.

A variety of shapes and sizes of elongate members and loops can be used, and these can both be optimized based on particular applications. The dimensions of the elongate member 12 and loop 22 will depend upon various factors, including the intended use of the wire guide and the vessels into which the wire guide will be positioned. For a wire guide intended to cannulate the common bile duct, suitable dimensions include a first diameter 16 of between approximately 0.0406cm (.016 inches) and approximately 0.0965cm (.038 inches), and preferably comprises a diameter of approximately 0.0889cm (.035 inches). The second diameter 20 of the wire guide preferably has a diameter of between approximately 0.0076cm (.003 inches) and approximately 0.0254cm (.010 inches), and preferably comprises a diameter of approximately 0.0152cm (.006 inches). The intermediate portion of this wire guide defines a taper between the first diameter 16 and the second diameter 20. The taper may be smaller or approximately the same size as the second diameter 20. Preferably, the intermediate portion defines a taper from approximately 0.0152cm (.006 inches) to approximately 0.0406cm (.016 inches). For this wire guide, the loop is preferably ovoid in shape with a length of between approximately 4 and approximately 5 millimeters, and a width of between approximately 2 and approximately 3 millimeters.

Figure 4 illustrates a wire guide 10 with a covering 30 is positioned over the closure member 24. The covering 30 can be polytetrafluoroethylene, or another suitable material. Examples of suitable coverings include fluoropolymers, polyurethanes, and other suitable coatings used in the medical device arts. Also, the covering 30 preferably is positioned over the closure member 24 and at least a section of the first portion 14. Particularly preferable, the covering is positioned over the first 32 and second 34 transition areas between the closure member 24 and the elongate member 12. This positioning of the covering 30 ensures a smooth surface at the transition areas 32, 34.

Alternatively, the covering 30 can comprise a coating on the elongate member 12. The coating is preferably applied to the entire elongate member 12, including the loop 22. Alternatively, the coating can be applied to only a portion of the elongate member. The coating may be applied by dipping, molding, or spraying a suitable coating material, such as polytecrafluoroethylene, urethane, and/or other polymeric coatings, directly to the elongate member 12.

A thin PTFE heat shrinkable material is a preferred coating. The heat shrinkable nature of these materials facilitate manufacturing while providing a lubricious coating, which facilitates navigation. In preferred embodiments, the thickness of the coating is between approximately 0.0025cm (0.001 inches) and 0.0254cm (0.010 inches). In particularly preferred embodiments, the thickness of the coating is between approximately 0.0025cm (0.001 inches) and 0.0127cm (0.005 inches). In still more preferred embodiments, the thickness of the coating is between approximately 0.0025cm (0.001 inches) and 0.0051 cm (0.002 inches). These preferred thicknesses provide suitable coatings while not adding significantly to the overall thickness of the device.

Also, the wire guide 10, with or without the covering 30, may be treated with a hydrophilic coating or hybrid polymer mixture, such as those based on polyvinyl puroladine and cellulose esters in organic solvent solutions. These solutions make the wire guide particularly lubricious when in contact with body fluids, which aids in navigation.

Radiopaque materials such as bismuth or gold can be added in the covering 30. Also, radiopaque markers known in the art can be placed on the elongate member 12, the loop 22, and/or the closure member 24. Several examples of suitable radiopaque materials and markers are known in the art, and any suitable material and/or marker can be utilized in the present invention.

As illustrated in the figures, the loop 22 is preferably formed by the elongate member 12. As an alternative, a separate member defining the loop can be affixed to a substantially straight elongate member to form the wire guide of the present invention. This may be advantageous when it is desirable to form the loop and elongate member of different materials. For example, a nylon or silicon loop could be formed and attached, such as by a closure member, to an elongate member formed of nitinol. Such an assembly could be coated and or associated with a covering as described above.

Figure 5 illustrates a wire guide 10 encountering an impediment 42 within a body vessel 40. As illustrated in the figure, the loop 22 deforms in response to its encounter with the impediment 42. Due to the presence of the loop 22 and closure member 24, the distal end 26 does not move relative to the remainder of the elongate member 12. Also, the loop 22 deforms in response to the impediment, enabling the wire guide to continue navigating along the interior of the vessel 40. The resiliency of the loop 22 creates a force opposing the impediment 42 and forces the loop 22 away from the impediment 42, which defines a path for the remainder of the wire guide 10 to follow.

Figure 6 illustrates a wire guide 10 encountering a torturous path 44 within a body vessel 40. As illustrated in the figure, the loop 22 deforms slightly in response to the torturous path 44. Also, due to the presence of the loop 22 and closure member 24, the distal end 26 does not move relative to the remainder of the elongate member 12. This allows the wire guide 10 to continue navigating along the interior of the body vessel 40. The taper of the intermediate region 21 provides additional flexibility to the wire guide 10, facilitating navigation of the loop 22 through the torturous path 44.

## Claims

1. A wire guide capable of manipulation about at least one of a tortuous path and an impediment comprising:
an elongate member (10) having a first portion (12) with a first diameter and a second portion (22) with a second diameter, the second diameter being smaller than the first diameter, the elongate member being bent back upon itself to form a loop having an interior space, and a closure member for maintaining the loop;
wherein the closure member comprises at least one attachment tab (25).

2. The wire guide of claim 1, further comprising a covering positioned over at least part of the first portion and the closure member.

3. The wire guide of claim 2, wherein the covering is a heat shrinkable polymeric coating.

4. The wire guide of any preceding claim, wherein a hydrophilic polymer coating lubricates an outer surface of the wire guide during navigation through a body lumen.

5. The wire guide of any preceding claim, wherein the second portion defines the loop.

6. The wire guide of any preceding claim, wherein the second portion defines only a portion of the loop.

7. The wire guide of any preceding claim, wherein the loop has a loop width that is greater than the first diameter.

8. The wire guide of any preceding claim, wherein the elongate member has an intermediate portion between the first portion and the second portion; and
wherein the intermediate portion defines a taper from the first diameter to the second diameter.

9. The wire guide of claim 8, wherein the loop is formed by disposing a distal end of the second portion adjacent to the intermediate portion.

10. The wire guide of any preceding claim, wherein the loop is formed by disposing a distal end of the second portion adjacent to a proximal portion of the second portion.

11. The wire guide of any preceding claim, further comprising a radiopaque marker positioned on the elongate member.

12. The wire guide of any preceding claim, further comprising a radiopaque marker positioned on the closure member.

13. The wire guide of one of claims 2 and 3, further comprising a radiopaque marker positioned on the covering.

14. The wire guide of any preceding claim, further comprising a radiopaque marker positioned on the loop.

15. The wire guide of any preceding claim, wherein the attachment tab comprises a first end that is fixedly connected to the second portion and a second end that is wrapped about the elongate member.

16. The wire guide of claim 15, further wherein the closure member comprises a pair of attachment tabs.

17. The wire guide of one of claims 15 and 16, wherein the second end of the attachment tab is wrapped about the second portion.

18. The wire guide of one of claims 15, 16, and 17, wherein the attachment tab and the second portion comprises a unitary construction.

## Patentansprüche

1. Führungsdraht zur Manipulation um mindestens einen gewundenen Pfad und Werkzeug, umfassend:
ein längliches Element (10) mit einem ersten Abschnitt (12) mit einem ersten Durchmesser und einem zweiten Abschnitt (22) mit einem zweiten Durchmesser, wobei der zweite Durchmesser kleiner ist als der erste Durchmesser, wobei das längliche Element zurückgebogen wird, um eine Schlaufe mit einem Innenraum zu bilden, und ein Verschlusselement zur Aufrechterhaltung der Schlaufe;
worin das Verschlusselement mindestens eine Befestigungslasche (25) umfasst.

2. Führungsdraht nach Anspruch 1, ferner eine Abdeckung umfassend, die zumindest über einem Teil des ersten Abschnitts und des Verschlusselements angeordnet ist.

3. Führungsdraht nach Anspruch 2, worin die Abdeckung eine aufschrumpfbare Polymer-Beschichtung ist.

4. Führungsdraht nach einem der vorhergehenden Ansprüche, worin eine hydrophile Polymer-Beschichtung bei der Navigation durch ein Körperlumen eine Außenfläche des Führungsdrahts schmiert.

5. Führungsdraht nach einem der vorhergehenden Ansprüche, worin der zweite Abschnitt die Schlaufe definiert.

6. Führungsdraht nach einem der vorhergehenden Ansprüche, worin der zweite Abschnitt nur einen Teil der Schlaufe definiert.

7. Führungsdraht nach einem der vorhergehenden Ansprüche, worin die Schlaufe eine Schlaufenbreite aufweist, die größer ist als der erste Durchmesser.

8. Führungsdraht nach einem der vorhergehenden Ansprüche, worin das längliche Element einen Zwischenabschnitt zwischen dem ersten Abschnitt und dem zweiten Abschnitt aufweist; und
worin der Zwischenabschnitt eine Verjüngung vom ersten Durchmesser zum zweiten Durchmesser definiert.

9. Führungsdraht nach Anspruch 8, worin die Schlaufe geformt wird, indem ein distales Ende des zweiten Abschnitts neben dem Zwischenabschnitt angeordnet wird.

10. Führungsdraht nach einem der vorhergehenden Ansprüche, worin die Schlaufe geformt wird, indem ein distales Ende des zweiten Abschnitts neben einem proximalen Abschnitt des zweiten Abschnitts angeordnet wird.

11. Führungsdraht nach einem der vorhergehenden Ansprüche, ferner eine röntgenopake Markierung umfassend, die auf dem länglichen Element angeordnet ist.

12. Führungsdraht nach einem der vorhergehenden Ansprüche, ferner eine röntgenopake Markierung umfassend, die auf dem Verschlusselement angeordnet ist.

13. Führungsdraht nach einem der Ansprüche 2 und 3, ferner eine röntgenopake Markierung umfassend, die auf der Abdeckung angeordnet ist.

14. Führungsdraht nach einem der vorhergehenden Ansprüche, ferner eine röntgenopake Markierung umfassend, die auf der Schlaufe angeordnet ist.

15. Führungsdraht nach einem der vorhergehenden Ansprüche, worin die Befestigungslasche ein erstes Ende umfasst, das fest mit dem zweiten Abschnitt verbunden ist, und ein zweites Ende, das um das längliche Element gewickelt ist.

16. Führungsdraht nach Anspruch 15, worin das Verschlusselement ferner ein Paar Befestigungslaschen umfasst.

17. Führungsdraht nach einem der Ansprüche 15 und 16, worin das zweite Ende der Befestigungslasche um den zweiten Abschnitt gewickelt ist.

18. Führungsdraht nach einem der Ansprüche 15, 16 und 17, worin die Befestigungslasche und der zweite Abschnitt einheitlich konstruiert sind.

## Revendications

1. Guide-fil capable d'être manipulé autour d'un chemin tortueux et ou d'un obstacle, comprenant :
un organe allongé (10) ayant une première portion (12) avec un premier diamètre et une deuxième portion (22) avec un deuxième diamètre, le deuxième diamètre étant plus petit que le premier diamètre, l'organe allongé étant replié sur lui-même pour former une boucle ayant un espace intérieur, et un organe de fermeture pour maintenir la boucle ;
l'organe de fermeture comprenant au moins une languette de fixation (25).

2. Guide-fil selon la revendication 1, comprenant en outre un recouvrement positionné par-dessus au moins une partie de la première portion et de l'organe de fermeture.

3. Guide-fil selon la revendication 2, dans lequel le recouvrement est un revêtement polymère thermorétractable.

4. Guide-fil selon l'une quelconque des revendications précédentes, dans lequel un revêtement polymère hydrophile lubrifie une surface externe du guide-fil pendant son passage à travers une lumière d'un corps.

5. Guide-fil selon l'une quelconque des revendications précédentes, dans lequel la deuxième portion définit la boucle.

6. Guide-fil selon l'une quelconque des revendications précédentes, dans lequel la deuxième portion définit seulement une portion de la boucle.

7. Guide-fil selon l'une quelconque des revendications précédentes, dans lequel la boucle a une largeur de boucle supérieure au premier diamètre.

8. Guide-fil selon l'une quelconque des revendications précédentes, dans lequel l'organe allongé a une portion intermédiaire entre la première portion et la deuxième portion ; et
dans lequel la portion intermédiaire définit un cône du premier diamètre au deuxième diamètre.

9. Guide-fil selon la revendication 8, dans lequel la boucle est formée en disposant une extrémité distale de la deuxième portion à côté de la portion intermédiaire.

10. Guide-fil selon l'une quelconque des revendications précédentes, dans lequel la boucle est formée en disposant une extrémité distale de la deuxième portion à côté d'une portion proximale de la deuxième portion.

11. Guide-fil selon l'une quelconque des revendications précédentes, comprenant en outre un marqueur radio-opaque positionné sur l'organe allongé.

12. Guide-fil selon l'une quelconque des revendications précédentes, comprenant en outre un marqueur radio-opaque positionné sur l'organe de fermeture.

13. Guide-fil selon l'une des revendications 2 ou 3, comprenant en outre un marqueur radio-opaque positionné sur le recouvrement.

14. Guide-fil selon l'une quelconque des revendications précédentes, comprenant en outre un marqueur radio-opaque positionné sur la boucle.

15. Guide-fil selon l'une quelconque des revendications précédentes, dans lequel la languette de fixation comprend une première extrémité qui est connectée de manière fixe à la deuxième portion et une deuxième extrémité qui est enroulée autour de l'organe allongé.

16. Guide-fil selon la revendication 15, dans lequel en outre l'organe de fermeture comprend une paire de languettes de fixation.

17. Guide-fil selon l'une des revendications 15 et 16, dans lequel la deuxième extrémité de la languette de fixation est enroulée autour de la deuxième portion.

18. Guide-fil selon l'une des revendications 15, 16 et 17, dans lequel la languette de fixation et la deuxième portion comprennent une construction unitaire.
